# EUROPEAN PATENT SPECIFICATION

(11) **EP 1 980 272 B1**
(45) Date of publication and mention of the grant of the patent: **08.07.2015**
(21) Application number: 08007211.9
(22) Date of filing: 11.04.2008
(51) Int. Cl.: A61K 47/10, A61K 9/20

(54) **Orally-disintegrating tablet and manufacturing method thereof**
Im Mund zerfallende Tablette und Herstellungsverfahren dafür
Comprimé se délitant en bouche et son procédé de fabrication

(30) Priority: 11.04.2007 JP 2007103925
(43) Date of publication of application: 15.10.2008
(73) Proprietor: NIPRO CORPORATION, Osaka-shi, Osaka 531-8510 (JP)
(72) Inventor: Sakuragi, Shiho, Kusatsu-shi Shiga-ken 525-0055 (JP); Yokoe, Jun-ichi, Kusatsu-shi Shiga-ken 525-0055 (JP); Katayama, Naohisa, Kusatsu-shi Shiga-ken 525-0055 (JP); Kai, Toshiya, Kusatsu-shi Shiga-ken 525-0055 (JP)
(74) Representative: Schüssler, Andrea

(56) References cited:
- WO-A1-2005/018617
- WO-A2-03/041683
- FR-A1- 2 790 387
- US-A- 5 576 014
- US-A1- 2002 071 864
- US-A1- 2007 281 009
- JETZER W E ET AL: "Comparison of two different experimental procedures for determining compaction parameters", INTERNATIONAL JOURNAL OF PHARMACEUTICS, ELSEVIER BV, NL, vol. 26, no. 3, 1 October 1985 (1985-10-01), pages 329-337, XP025524663, ISSN: 0378-5173, DOI: 10.1016/0378-5173(85)90241-8 [retrieved on 1985-10-01]
- HWANG R ET AL: "Effect of binder and geometry of tablet on rate of wear, hardness and tensile strength", DRUG DEVELOPMENT AND INDUSTRIAL PHARMACY, NEW YORK, NY, US, vol. 19, no. 5, 1 January 1993 (1993-01-01), pages 507-519, XP009087032, ISSN: 0363-9045, DOI: 10.3109/03639049309062963
- JOSHI, A A; DURIEZ, X: "Added Functionality Excipients: An ANswer to Challenging Formulations", PHARMACEUTICAL TECHNOLOGY, 1 January 2004 (2004-01-01), pages 12-16, Retrieved from the Internet: URL:http://www.roquette-pharma.com/media/d eliacms/media//2/277-6dcdd1.pdf>

## Description

### BACKGROUND OF THE INVENTION

### 1. Field of the Invention

The present invention relates to an orally-disintegrating tablet and a manufacturing method thereof, and more specifically relates to an orally-disintegrating tablet that disintegrates easily in the buccal cavity even though it has comparatively high hardness and a manufacturing method thereof.

### 2. Background Information

Orally-disintegrating tablets are formulations that are easily taken by elderly people, children and patients that have difficulty swallowing. They are attracting attention as formulations that improve quality of life (QOL) because administration without water is possible.

Typically, the orally-disintegrating tablets disintegrate rapidly after administration, but there is a problem in that they are difficult to use with automatic packaging machines due to their poor mechanical strength.

In addition, in the manufacturing methods therefore, a method of pouring a dispersion liquid of a drug substance and an additive into PTP pockets or the like and lyophilizing them; a method using special machines such as a wet tableting machine; a method of tableting noncrystalline sugars at low pressure and humidifying and drying them to increase the hardness, and the like are known, but all of these methods are troublesome and complex and invite increases in production costs. Also, if the mechanical strength of the orally-disintegrating tablets themselves, which are obtained in this manner, is increased comparatively in consideration of handling properties, the property of disintegrating in the mouth is reduced, and a sufficiently satisfactory product is not obtained.

Typically, sugar alcohols, which have good disintegration properties are used in a suitable manner as additives in orally-disintegrating tablets. However, sugar alcohols are normally in the form of fine powders, have poor molding properties and cannot be molded by tableting without modification. In addition, assuring tableting properties by mixing cellulose based excipients, such as crystal cellulose, into the sugar alcohol has been proposed (see, for example, Japanese Laid-Open Patent Application 2004-175796), but if the cellulose component is contained in large quantities, fiber remains when it is administered, and the feeling on the tongue and taste degrade. Furthermore, tableting is possible using granulated sugar alcohols, but the disintegration properties in the buccal cavity deteriorate, and tablets that require a minute or more to disintegrate are formed.

FR 2 790 387 A1, US 2002/071864 A1, WO 2005/018617 A1, and US 2007/281009 A1 describe tablets comprising active substances and sugar alcohol powders and granules, respectively.

### SUMMARY OF THE INVENTION

In light of the problems described above, it is an object of the present invention to provide an orally-disintegrating tablet that may be formed using a normal tableting machine, that reduces the disintegration time in the buccal cavity while imparting a hardness that is the same as an ordinary tablet and that has a suitable mechanical strength, and an manufacturing method thereof.

The inventors has found, as a result of earnest investigations into additives for conventional orally-disintegrating preparations, that it is possible to unexpectedly obtain more satisfactory characteristics, by using a single ingredient in which forms are different, for both of the characteristics in the trade-off relationship between improving the mechanical strength of the orally-disintegrating tablets and the disintegration properties in the buccal cavity by mixing a granulation of sugar alcohol, which is superior in molding properties but degrades the disintegration properties, into a powder sugar alcohol, which has superior disintegration properties but degrades the molding properties.

The present invention provides a orally-disintegrating tablet comprising a principal agent, a D-mannitol powder and a sugar alcohol granule, wherein the orally-disintegrating tablet has a hardness of 30 N or greater. The orally-disintegrating tablet is defined in claim 1, while preferred embodiments are defined in the dependent claims. Further, the present invention provides a manufacturing method for an orally-disintegrating tablet comprising:
mixing D-mannitol as a sugar alcohol powder and a sugar alcohol granule with a principal agent and D-mannitol powder as defined in claim 7 to obtain a mixture and compression-molding the mixture at a hardness of 30 N or greater.

According to the present invention, a smooth swallowing orally-disintegrating tablet, for which both the mechanical strength and the disintegration properties in the buccal cavity are favorable with using a normal tableting machine and without using special devices and the feeling on the tongue and taste are favorable, and a manufacturing method thereof are provided.

### DETAILED DESCRIPTION OF THE PREFERRED EMBODIMENTS

An orally-disintegrating tablet of the present invention contains at least a principal agent and a sugar alcohol.

There is no particular restriction on the principal agent here, and examples of the principal agent include medicines for motion sickness, analgesic antipyretic, aromatic stomachic, digestant, antacid agent, vitamin, nutrient tonic, enzyme preparation, nutrient tonic supplement, anti-inflammatory, antirheumatic drug, gout remedy, antihistamine, allergy agent, antibiotic agent, synthetic antibacterial, medicine for dental and oral use, bronchodilator, cough remedy, expectorant drug, sleep sedative, anxiolytic agent, antiepileptic drug, psychoneurotic agent, autonomic agent, central nervous system drug, antispasmodic agent, ameliorant of cerebral metabolism, ameliorant of cerebral circulation, antiParkinson's disease agent, Alzheimer therapeutic agent, cardiotonic agent, antiarrhythmia agent, diuretic agent, vasoconstrictor, vasodilatation agent, hypotensive agent, antihyperlipemia agent, constipating agent, peptic ulcer agent, cathartic, hormone agent, diabetes agent, as well as prodrugs.

The primary agent may have any form, powder, solid, granulated powder or the like. There is no particular restriction on the size, and it may be suitably prepared in consideration of the feel on the tongue when administered as an orally-disintegrating tablet. Moreover, a method for making the grain diameters uniform such as a sieve or membrane filter and a method for pulverizing, such as a ball mill pulverizer, hammer mill pulverizer, or pin mill pulverizer may cited for giving the principal agent a suitable shape and size. In addition, it is suitable for the principal agent to be about 0.1 % or more by weight, and more suitably about 1 % or more by weight, as well as about 50 % or less by weight of the total weight of the orally-disintegrating tablet.

Examples of the sugar alcohol include D-mannitol, erythritol, sorbitol, xylitol, trehalose, maltitol and lactol. Among these, it is preferably D-mannitol, erythritol, sorbitol, and xylitol. These can be used alone or as mixture of two or more.

The orally-disintegrating tablet of the present invention is formed using at least both a D-mannitol powder as a powder sugar alcohol and a granulated sugar alcohol. Powder means that it is not in a granulated form. Normally, the average particle size in powder sugar alcohols is cited as being smaller than 50 µm, and typically, use of ones about 40 µm or less is preferable.

The granule is a sugar alcohol granulated by a method commonly known in the field, and for example, either wet granulation or dry granulation may be used for the manufacturing method. In the case of wet granulation, granulation may be done by a variety of devices, such as a fluid bed granulator and dryer, agitating granulator, cylindrical extrusion granulator, rolling fluid bed granulation and coating machine, or spray drying. In the case of dry granulation, granulation may be done using various devices, dry granulators, such as roller compactors and slug tableting machines. Among these, forming using a wet granulation method is preferable. Since wet granulation methods yield more uniform particle diameters than dry granulation methods, and the proportion of fine powder is reduced, a granulated powder with better molding properties may typically be obtained.
Moreover, when the sugar alcohol is granulated, any additives that may be added for pharmaceutical products, such as sweeteners, disintegration agents and colorants, may be added, as will be discussed in the following.

It is preferable for the granulated substance to have, for example, an average particle diameter of 80 µm or greater, and 500 µm or less, 400 µm or less, 300 µm or less, 200 µm or less, 150 µm or less. Particularly, it has an average particle diameter of at least 80 µm. From another point of view, it is suitable for the granulated substance to have a particle diameter of 2 to 20 times, preferably 3 to 8 times that of the powder sugar alcohol.

It is suitable for the mixing ratio for the powder sugar alcohol and the granulated sugar alcohol to be a ratio by weight of 4:1 to 1:1 for the sugar alcohols. This is because molding becomes difficult for the tablets if there is too much of the powder, and if there is too much of the granulated substance, the orally-disintegration time tends to increase. Because of the mixing of the powder sugar alcohol and the granulated sugar alcohol in this range, there is a difference from a comparatively low density tablet, where the voids between the sugar alcohol particles like those in an orally-disintegrating tablet formed with granulated sugar alcohol only are connected, in that the voids between the particles are present independently to a moderate degree, and the powder sugar alcohol is dispersed in the vicinity of these voids. While molding with comparatively high hardness is possible because of this particle distribution, the tablet can easily be disintegrated in a short period of time by the saliva in the buccal cavity. Moreover, its is preferable for the independent voids here to be at least the same as the average particle diameter of the granulated sugar alcohol or to be voids with smaller diameters than these, and it is preferable for these independent voids be present to a degree of 50% or more, 60 % or more, and 70 % or more of the total void volume.

In addition, in the present invention, it is suitable for the sugar alcohol powder and the sugar alcohol granules to be 50 % or more, 55 % or more, 60 % or more by weight of the total weight of the orally-disintegrating preparation. There is no particular upper restriction, but, normally it is about 70 % by weight. By including the sugar alcohols in this range, it is possible to assure both the disintegration properties and molding properties with the single sugar alcohol component, and along with disintegration happening smoothly in the buccal cavity while there is suitable mechanical strength, it is possible to make it easy to swallow with a good feeling on the tongue, feeling of roughness and flavor in the buccal cavity.

Furthermore, normally used additives and the like may be included in the orally-disintegrating tablet of the present invention. Examples of the additive include disintegrating agent, excipient, binder, solubilizing agent, lubricant, fluidiser, sweetener, fragrance, foaming agent, surfactant, pH adjuster, preservative and colorant.

Examples of the disintegrating agent include crospovidone, sodium starch glycolate, sodium carboxymethyl starch, starch, partially pregelatinized starch, cornstarch, lactose, calcium carbonate, precipitated calcium carbonate, calcium citrate, light silicic anhydride, synthetic aluminum silicate crystalline cellulose, low substitution degree hydroxypropylcellulose, croscarmellose, sodium croscarmellose, calcium carboxymethylcellulose, carmellose and hydroxypropyl starch. These can be used alone or as mixture of two or more. Among these, it is preperably croscarmellose, sodium starch glycolate, carmellose, starch, hydroxypropyl starch and crospovidone. The amount of the disintegrating agent can be, for example, included 0.1 % or more, preferably 0.5 % or more, and more preferably 2 % or more by weight with respect to the total weight of the orally-disintegrating tablet. Also, it can be included 30 % or less, preferably 25 % or less, and more preferably 15 % by weight.

Examples of the excipient include glucose, fructose, lactose, sucrose and hydrogenated maltose. These can be used alone or as mixture of two or more.

Examples of the binder include a water-soluble substance such as gelatine, agar, alginic acid, sodium alginate, dextrin, xanthan gum, arabian gum, hydroxyethylcellulose, hydroxypropylcellulose, hydroxypropylmethylcellulose, polyvinylpyrrolidone, partially saponification polyvinyl alcohol, methylcellulose, pullulan, partially pregelatinized starch and sugar. These can be used alone or as mixture of two or more.

Examples of the solubilizing agent include magnesium oxide, calcium oxide, sodium citrate, magnesium chloride, sodium carbonate and sodium bicarbonate. These can be used alone or as mixture of two or more.

Examples of the lubricant include magnesium stearate, calcium stearate, talc, sucrose fatty acid ester, polyethyleneglycol, stearic acid, light silicic anhydride, hydrogenated rape oil, hydrogenated castor oil, glycerin fatty acid ester, sodium stearyl fumarate, sodium benzoate, L-leucin and L-valine. These can be used alone or as mixture of two or more.

Examples of the fluidizer include hydrated silicon dioxide and light silicic anhydride.

Examples of the sweetener include aspartame, sodium saccharin, dipotassium glycyrrhizinate, stevia and thaumatin. These can be used alone or as mixture of two or more.

Examples of the fragrance include mint, lemon or orange extract.

Examples of the foaming agent include tartrate, citrate and bicarbonate.

Examples of the surfactant include an anionic surfactant such as sodium alkylsulfate; a nonionic surfactant such as sucrose fatty acid ester, polyoxyethylene sorbitan fatty acid ester, polyoxyethylene fatty acid ester and polyoxyethylene castor oil derivatives. These can be used alone or as mixture of two or more.

Examples of the pH adjuster include an organic acid such as glycin, sodium acid carbonate, dibasic calcium phosphate, dibasic sodium phosphate, acetic acid, succinic acid, tartaric acid, fumaric acid, and citric acid.

Examples of the preservative include benzoic acid, parahydroxybenzoic acid or a salt thereof.

Examples of the colorant include yellow oxide of iron, yellow iron sesquioxide, iron sesquioxide (red), orange essence, brown iron oxide, caramel, light silicic anhydride, Food Blue No.5, Food Yellow No.4, Food Yellow No.4 aluminum lake, Food Yellow No.5, Food Red No.2 (Amaranth), Food Red No.3, Food Red No.102, talc, sodium tetrafluorescein, green tea powder and Vitamin C.

It is preferable for the orally-disintegrating tablet of the present invention to have a hardness that does not allow it to be destroyed by being removed from a packaging container or the like. Specifically, it is suitably 30 N or greater, preferably 35 N or greater, more preferably 40 N or greater. The hardness of preparations may be measured using a tablet hardness gauge (Schleuinger Inc., Tablet Tester 6D), for example.

There is no particular restriction on the shape of the orally-disintegrating preparation of the present invention, and it may be made into a disk shape, donut shape, polygonal disk shape, spherical shape, elliptical shape or capsule shape. Among these, the disk shape, which is the normal tablet shape, is preferable. There is no particular restriction on the size, and it is suitable for it to be slightly large so that it cannot be directly swallowed. For example, a diameter of 3 to 30 mm and a thickness of 1 to 10 mm are preferable.

In addition, the manufacturing method for the orally-disintegrating tablet of the present invention comprises mixing the sugar alcohol powder and sugar alcohol granules into the principal agent and molding.

The mixing of the principal agent, sugar alcohol powder, sugar alcohol granules and optionally an additive described above may be carried out using commonly known devices by a method commonly known in the field. The principal agent and the sugar alcohol powder are normally not ones that have been granulated, and the sugar alcohol granules have been granulated in advance as described above, at mixing. These may be mixed in any order or at the same time.

The mixing at this time is preferably carried out without wetting or adding moisture. Here, wetting or adding moisture means water content of more than 5% of the total weight of the orally-disintegrating tablet. Therefore, without wetting and without the addition of moisture means mixing water of 5% or less to the total weight of the orally-disintegrating tablet.

It is suitable to use commonly known devices or the like, compress and carry out the molding of the mixture by methods that are commonly known in the field. For example, a tableting mortar and upper and lower pestles for tableting are used, and a hydraulic hand press, a single punch tableting machine, a rotary tableting machine or the like is used for the device for compression molding.

The tablets obtained in compression molding have a suitable hardness, for example 30 N or greater, and suitable independent voids are assured in the tablets. Adjustment and execution so that there can be rapid disintegration are necessary for the orally-disintegrating tablets. For example, there is no particular restriction for the tableting pressure, and it may be adjusted suitably according to the device used, principles, size of tablet and type of principal agent. When a device like that described above is used, for example, a tableting pressure of 50 kg/cm² or greater and 1500 kg/cm² or less is suitable, and 300 kg/cm² or greater and 1000 kg/cm² or less is preferable.

Moreover, it is also suitable for being carried out without wetting and without moisture being added at compression molding.

Typically, with molding as a production method for orally-disintegrating tablets, there is a method of drying after the mixed powder is tableted in a wet state (wet tableting), a method of mixing in a noncrystalline sugar in advance, tableting at a comparatively low pressure, adding moisture and drying (aging) after that to increase the tablet hardness. These methods may be used in the present invention, but as was described earlier, in molding methods for normal tablets, there is merit in being able to manufacture them conveniently without requiring special processes like these.

In the following, examples of the orally-disintegrating tablet of the present invention and the manufacturing method thereof will be described in detail.

### Example 1

500 g of D-mannitol was introduced into a fluid bed granulator (Powerex Corp., Multiplex), a 10% aqueous solution of D-mannitol (Nikken Fine Chemical Co., Ltd.) sprayed as the spraying fluid and fluid bed granulation carried out. After 500g was sprayed, it was dried and granulated mannitol was obtained (average particle diameter 150 to 220 µm).

In addition, using the same method, 500 g of erythritol (Mitsubishi Chemical Corp.) was introduced into, a 10% aqueous solution of erythritol sprayed as the spray fluid, fluid bed granulated, sprayed 500 g of the aqueous solution, after that dried, and granulated erythritol obtained (average particle diameter 80 to 130 µm).

For the commercial sugar alcohol granules, direct tableting mannitol (Patikku 100M, Merck, granulated mannitol, average particle diameter 90 to 120 µm) was used for the granulated substance.

Crystal cellulose (Asahi Kasei Chemicals Corp., Seorasu PH301), crospovidon XL10 (ISP), aspartame (Ajinomoto Co., Inc), magnesium stearate (Taihei Chemical Industrial Co., Ltd.), crosscarmellose (Asahi Kasei Chemicals Corp.) and hydroxypropyl starch (Fruend Industrial Co., Ltd.) were used as additives.

The various compounds described above were tableted so as to have a diameter of 7 mm with a weight of 100 mg per tablet in a rotary tableting machine according to the recipe given in Table 1 through Table 3.

In addition, as a comparative example, an orally-disintegrating preparation was tableted using only the powder sugar alcohol or only the granulated substance as shown in Table 4.

**Table 1**

| Example | 1 | 2 | 3 | 4 | 5 | 6 | 7 | 8 | 9 | 10 |
|---|---|---|---|---|---|---|---|---|---|---|
| D-mannitol | 37.5 | 45 | 50 | 37.5 | 45 | 50 | 56.3 | 37.5 | 45 | 50 |
| Granulated Mannitol | 37.5 | 30 | 25 | | | | | | | |
| Direct Tableting Mannitol | | | | 37.5 | 30 | 25 | 18.7 | | | |
| Granulated Erythritol | | | | | | | | 37.5 | 30 | 25 |
| Crystal Cellulose | 13 | 13 | 13 | 13 | 13 | 13 | 13 | 13 | 13 | 13 |
| Crospovidon | 10 | 10 | 10 | 10 | 10 | 10 | 10 | 10 | 10 | 10 |
| Aspartame | 1 | 1 | 1 | 1 | 1 | 1 | 1 | 1 | 1 | 1 |
| Magnesium Stearate | 1 | 1 | 1 | 1 | 1 | 1 | 1 | 1 | 1 | 1 |
| Tableting Pressure (kgf/cm²) | 450 | 700 | 850 | 450 | 650 | 800 | 950 | 500 | 750 | 900 |
| Hardness (N) | 40 | 40 | 35 | 40 | 45 | 35 | 30 | 40 | 40 | 30 |
| Disintegration Time (s) | 28 | 20 | 15 | 25 | 15 | 13 | 10 | 24 | 18 | 11 |

**Table 2**

| Example | 11 | 12 | 13 | 14 |
|---|---|---|---|---|
| D-mannitol | 45 | 45 | 45 | 45 |
| Direct Tableting Mannitol | 30 | 30 | 30 | 30 |
| Crystal Cellulose | 13 | 13 | 13 | 8 |
| Crospovidon | 2 | 5 | | |
| Crosscarmellose | | | 5 | 5 |
| Hydroxypropyl Starch | 8 | 5 | 5 | 10 |
| Aspartame | 1 | 1 | 1 | 1 |
| Magnesium Stearate | 1 | 1 | 1 | 1 |
| Tableting Pressure (kgf/cm²) | 500 | 650 | 700 | 800 |
| Hardness (N) | 45 | 40 | 37 | 40 |
| Disintegration Time (s) | 25 | 19 | 27 | 24 |

**Table 3**

| Example | 15 | 16 | 17 | 18 |
|---|---|---|---|---|
| Acetaminophen | 15 | | | |
| Risperidone | | | 2 | |
| Zolpidem tartrate | | 10 | | |
| Amlodipine Besilate | | | | 3.47 |
| D-mannitol | 36 | 39 | 45 | 43.53 |
| Granulated Mannitol | 24 | 26 | 28 | 28 |
| Direct Tableting Mannitol | | | | 37.5 |
| Granulated Erythritol | | | | |
| Crystal Cellulose | 13 | 13 | 13 | 13 |
| Crospovidon | 10 | 10 | 10 | 10 |
| Aspartame | 1 | 1 | 1 | 1 |
| Magnesium Stearate | 1 | 1 | 1 | 1 |
| Tableting Pressure (kgf/cm²) | 800 | 750 | 650 | 650 |
| Hardness (N) | 35 | 40 | 45 | 40 |
| Disintegration Time (s) | 28 | 20 | 15 | 18 |

**Table 4**

| Comparative Example | 1 | 2 |
|---|---|---|
| D-mannitol | 75 | |
| Granulated Mannitol | | |
| Direct Tableting Mannitol | | 75 |
| Granulated Erythritol | | |
| Crystal Cellulose | 13 | 13 |
| Crospovidon | 10 | 10 |
| Aspartame | 1 | 1 |
| Magnesium Stearate | 1 | 1 |
| Tableting Pressure (kgf/cm²) | 1000 | 600 |
| Hardness (N) | Tableting was impossible | 40 |
| Disintegration Time (s) | | 45 |

From the results in Tables 1 through 4, the hardness was 30 N or greater for all of the examples of the present invention, but even though it was high, the buccal cavity disintegration time was within 30 sec., and a rapidly disintegrating orally-disintegrating tablet was obtained. Moreover, all of the orally-disintegrating tablets in the examples had a good feel on the tongue in the buccal cavity, and there was easy swallowing with no feeling of roughness or sense of discomfort.

On the other hand, when only the powder sugar alcohol was used in the comparative examples, tableting was impossible, and tablets could not be formed. In addition, when only the granulated sugar alcohol was used, the buccal cavity disintegration time increased.

Regardless of the type of principal agent and regardless of the part that is disintegrated, the present invention may be used over a wide range of formulations that disintegrate and administer the agent.
This application claims priority to Japanese Patent Application No. 2007-103925. The entire disclosure of Japanese Patent Application No. 2007-103925.

## Claims

1. An orally-disintegrating tablet comprising a principle agent, a D-mannitol powder and a sugar alcohol granule, wherein the orally-disintegrating tablet has a hardness of 30 N or greater which is measured using a tablet hardness gauge, wherein the average particle size in the D-mannitol powder is smaller than 50 µm and the average particle size in the sugar alcohol granulate is at least 80 µm, wherein the principal agent and D-mannitol powder are not ones that have been granulated, the mixing ratio for the D-mannitol powder and the sugar alcohol granule is a ratio by weight of 4:1 to 1:1 and the hardness is obtained by compression molding.

2. The orally-disintegrating tablet according to claim 1, wherein the sugar alcohol granule has a particle diameter of 2 to 20 times that of the D-mannitol powder.

3. The orally-disintegrating tablet according to claim 1, wherein the sugar alcohol granule is formed by a wet granulation method.

4. The orally-disintegrating tablet according to claim 3, wherein the sugar alcohol granule is formed by a method of agitating and granulating, fluid bed granulating or spray drying.

5. The orally-disintegrating tablet according to claim 1, wherein an amount of the D-mannitol powder and the sugar alcohol granule is 50 % or more by weight of the total weight of the orally-disintegrated tablet.

6. The orally-disintegrating tablet according to claim 1, further comprising at least one compound selected from the group consisting of croscarmellose, sodium starch glycolate, carmellose, starch, hydroxypropyl starch and crospovidone.

7. A manufacturing method for an orally-disintegrating tablet comprising:
- mixing a D-mannitol powder having an average particle size of smaller than 50 µm and a sugar alcohol granule having an average particle size of at least 80 µm with a principle agent and D-mannitol powder that are not ones that have been granulated to obtain a mixture, wherein the mixing ratio for the D-mannitol powder and the sugar alcohol granule is a ratio by weight of 4:1 to 1:1; and
- compression molding the mixture at a hardness of 30 N or greater which is measured using a tablet hardness gauge.

8. The method according to claim 7, wherein the mixing and compression-molding are carried out without wetting and without addition of moisture.

## Patentansprüche

1. Schmelztablette umfassend einen Hauptwirkstoff, ein D-Mannitol-Pulver und ein Zuckeralkoholgranulat, wobei die Schmelztablette eine Härte von 30 N oder mehr aufweist, was unter Verwendung eines Tablettenhärtemessgeräts gemessen wird, wobei die durchschnittliche Partikelgröße im D-Mannitol-Pulvers geringer als 50 µm ist und die durchschnittliche Partikelgröße im Zuckeralkoholgranulat mindestens 80 µm ist, wobei der Hauptwirkstoff und das D-Mannitol-Pulver nicht von der Art sind, dass sie granuliert worden sind, das Mischverhältnis für das D-Mannitol-Pulver und das Zuckeralkoholgranulat in einem Gewichtsverhältnis von 4:1 zu 1:1 liegt und die Härte mittels Formpressen erhalten wird.

2. Schmelztablette gemäß Anspruch 1, wobei das Zuckeralkoholgranulat einen 2- bis 20-fachen Partikeldurchmesser als der vom D-Mannitol-Pulver aufweist.

3. Schmelztablette gemäß Anspruch 1, wobei das Zuckeralkoholgranulat mittels eines Nassgranulierongsverfahrens gebildet wird.

4. Schmelztablette gemäß Anspruch 3, wobei das Zuckeralkoholgranulat mittels eines Rühr- und Granulierungs-, Wirbelschichtgranulierungs- oder Sprühtrocknungsverfahrens gebildet wird.

5. Schmelztablette gemäß Anspruch 1, wobei die Menge vom D-Mannitol-Pulver und vom Zuckeralkoholgranulat 50 Gew.-% oder mehr vom Gesamtgewicht der Schmelztablette ausmachen.

6. Schmelztablette gemäß Anspruch 1, weiterhin mindestens eine Verbindung, ausgewählt aus der Gruppe bestehend aus Croscarmellose, Natriumstärkeglykolat, Carmellose, Stärke, Hydroxypropylstärke und Crospovidon, umfassend.

7. Hersteilverfahren für eine Schmelztablette umfassend:
- Mischen eines D-Mannitol-Pulvers mit einer durchschnittlichen Partikelgröße von weniger als 50 µm und einem Zuckeralkolholgranulat mit einer durchschnittlichen Partikelgröße von mindestens 80 µm, mit einem Hauptwirkstoff und D-Mannitol-Pulver, die nicht von der Art sind, dass sie granuliert worden sind, um eine Mischung zu erhalten, wobei das Mischverhältnis für das D-Mannitol-Pulver und das Zuckeralkoholgranulat in einem Gewichtsverhältnis von 4:1 bis 1:1 liegt; und
- Formpressen der Mischung bei einer Härte von 30 N oder mehr, was unter Verwendung eines Tablettenhärtemessgeräts gemessen wird.

8. Verfahren gemäß Anspruch 7, wobei das Mischen und das Formpressen ohne Befeuchtung und ohne Feuchtigkeitszugabe durchgeführt werden.

## Revendications

1. Comprimé se délitant en bouche, comprenant un agent principal, une poudre de D-mannitol et un granulé d'alcool de sucre, dans lequel le comprimé se délitant en bouche présente une dureté de 30 N ou supérieure qui est mesurée à l'aide d'une jauge de dureté de comprimé, dans lequel la grosseur de particule moyenne de la poudre de D-mannitol est inférieure à 50 µm et la grosseur de particule moyenne dans le granulé d'alcool de sucre est d'au moins 80 µm, dans lequel l'agent principal et la poudre de D-mannitol ne sont pas ceux qui ont été granulés, le rapport de mélange de la poudre de D-mannitol et du granulé d'alcool de sucre est un rapport en poids de 4:1 à 1:1 et la dureté est obtenue par moulage par compression.

2. Comprimé se délitant en bouche selon la revendication 1, dans lequel le granulé d'alcool de sucre présente un diamètre de particule de 2 à 20 fois celui de la poudre de D-mannitol.

3. Comprimé se délitant en bouche selon la revendication 1, dans lequel le granulé d'alcool de sucre est formé par un procédé de granulation par voie humide.

4. Comprimé se délitant en bouche selon la revendication 3, dans lequel le granulé d'alcool de sucre est formé par un procédé d'agitation et de granulation, de granulation en lit de fluide ou de séchage par pulvérisation.

5. Comprimé se délitant en bouche selon la revendication 1, dans lequel une quantité de la poudre de D-mannitol et du granulé d'alcool de sucre est de 50% ou plus en poids du poids total du comprimé se délitant en bouche.

6. Comprimé se délitant en bouche selon la revendication 1, comprenant par ailleurs au moins un composé choisi parmi le groupe composé de croscarmellose, de glycolate d'amidon sodique, de carmellose, d'amidon, d'amidon hydroxypropylique et de crospovidone.

7. Procédé de fabrication d'un comprimé se délitant en bouche, comprenant le fait de:
- mélanger une poudre de D-mannitol présentant une grosseur de particule moyenne de moins de 50 µm et un granulé d'alcool de sucre présentant une grosseur de particule moyenne d'au moins 80 µm avec un agent principal et de la poudre de D-mannitol qui ne sont pas ceux qui ont été granulés pour obtenir un mélange, où le rapport de mélange de la poudre de D-mannitol et du granulé d'alcool de sucre est un rapport en poids de 4:1 à 1:1; et
- mouler par compression le mélange à une dureté de 30 N ou supérieure qui est mesurée à l'aide d'une jauge de dureté de comprimé.

8. Procédé selon la revendication 7, dans lequel le mélange et le moulage par compression sont réalisés sans mouillage et sans addition d'humidité.
